# EUROPEAN PATENT APPLICATION

(11) **EP 3 482 731 A1**
(43) Date of publication of application: **15.05.2019**
(21) Application number: 17823876.2
(22) Date of filing: 22.05.2017
(51) Int. Cl.: A61F 13/536, A61F 13/49, A61F 13/53, A61F 13/534, A61F 13/535

(54) **ABSORBENT ARTICLE**

(30) Priority: 08.07.2016 JP 2016136351
(71) Applicant: Unicharm Corporation, Ehime 799-0111 (JP)
(72) Inventor: ONISHI, Kazuaki, Kanonji-shi Kagawa 769-1602 (JP)
(74) Representative: Staeger & Sperling Partnerschaftsgesellschaft mbB
(86) International application number: PCT/JP2017/019064
(87) International publication number: WO 2018/008268

(57) **Abstract**

The present disclosure provides an absorbent article that has an excellent ability to diffuse bodily fluid, an excellent rate of absorption, and an excellent ability to prevent the fluid from returning even after repeatedly absorbing bodily fluid. This absorbent article is provided with an absorbent core (9) comprising superabsorbent polymer particles. The absorbent core (9) comprises a first layer (6) on the non-skin facing side and a second layer (8) on the skin facing side. The first layer (6) is provided with: groove parts (18) including a main groove part (19) that extends along the longitudinal direction L and passes therethrough in the thickness direction T; and base parts (20) extending along the longitudinal direction L. The main groove parts (19) and the base parts (20) alternately extend in the lateral direction W. The second layer (8) is provided with main groove part corresponding parts (22) and base part corresponding parts (24) at positions overlapping the main groove parts (19) and the base parts (20) in the thickness direction T. The superabsorbent polymer particles (32) in the base parts (20) and the main groove corresponding parts (22) have a smaller average density than the superabsorbent polymer particles (32) in the base part corresponding parts (24).

## Description

### FIELD

The present disclosure relates to an absorbent article, and especially to a disposable diaper.

### BACKGROUND

In an absorbent article, various improvements are being made in order to realize excellent comfortable feeling while preventing leakage of excrement such as urine, etc.

For example, in Patent Document 1, an absorbent article is disclosed which includes a top sheet disposed on the skin contact surface side, a back sheet disposed on the non - skin contact surface side, and an absorbent body disposed between the both sheets, wherein the absorbent body has a longitudinal direction and a width direction orthogonal to the longitudinal direction, the absorbent body includes protruded absorbent portions which are present in a discontinuous and scattered manner in the planar direction on the non - skin contact surface side of the excretory portion corresponding region, and a liquid flowing structure which is disposed in each of the longitudinal direction and width direction in between the protruded absorbent portions, the liquid flowing structure is configured by a recessed portion which is recessed in a grooved manner from the non - skin contact surface side of the absorbent body in the thickness direction, and a recessed portion absorbent portion which is positioned in the bottom portion on the skin contact surface side of the recessed portion, a density of the recessed portion absorbent portion is smaller than a density of the protruded absorbent portions, and an area of a surface on an upper side on the skin contact surface side of the protruded absorbent portions is larger than an area of a surface on a lower side on the non - skin contact surface side of the protruded absorbent portions.

### [PRIOR ART DOCUMENTS]

### [PATENT DOCUMENTS]

[Patent Document 1] Japanese Unexamined Patent Publication No. 2012 - 130365

### DISCLOSURE OF THE INVENTION

### [PROBLEMS TO BE SOLVED BY THE INVENTION]

In the absorbent article described in Patent document 1, since it is easy for the body fluid which has reached the recessed portion absorbent portion or the recessed portion to be transferred and retained in the adjacent protruded absorbent portions, in the case when the amount of the body fluid is small, it is easy to secure the absorbency. However, after a certain amount of the body fluid is absorbed, especially when after the body fluid is absorbed for a plurality of times, the absorbent body is swollen by the absorbed body fluid and the width of the recessed portion is to be narrower, and in some cases, the recessed portion almost disappears, whereby there has been a fear that the absorbency and diffusibility of the body fluid are deteriorated. Further, in the absorbent article described in Patent document 1, since the protruded absorbent portions which have smaller density than the recessed portion absorbent portion are present in a discontinuous and scattered manner in the planar direction, there was a fear that the body fluid which has been transferred to the protruded absorbent portions by the capillary phenomenon stays within the protruded absorbent portions, which makes it difficult for the body fluid to be diffused over the entire absorbent body. As a result, in the absorbent article described in Patent document 1, especially when after the body fluid is absorbed for a plurality of times, there have been cases in which the body fluid which can no longer be retained in the protruded absorbent portions is concentrated in the vicinity of the excretory portion corresponding region, whereby the repeated absorbing function and the liquid return suppression function of the absorbent body are deteriorated.

Accordingly, the object of the present disclosure is to provide an absorbent article which is excellent in the diffusibility, the absorbing speed, and the liquid return suppression of the body fluid even when the body fluid is repeatedly absorbed.

### [MEANS FOR SOLVING THE PROBLEMS]

The inventors of the present invention found out that an absorbent article comprising an absorbent core which includes a longitudinal direction, a short length direction, and a thickness direction, and further includes super absorbent polymer particles, wherein
the absorbent core includes a non - skin facing surface and a skin facing surface, and is formed by including a first layer which is present on a non - skin facing surface side and a second layer which is present on a skin facing surface side,
the first layer includes a plurality of groove portions including a plurality of main groove portions which extend along the longitudinal direction and penetrates the first layer in the thickness direction; and a plurality of base portions which extend along the longitudinal direction, and each of the plurality of main groove portions and each of the plurality of base portions extend alternately in the short length direction,
the second layer includes a plurality of main groove portion corresponding portions and a plurality of base portion corresponding portions at positions overlapping with the plurality of main groove portions and the plurality of base portions, respectively, in the thickness direction, and
both an average density of the super absorbent polymer particles included in each of the plurality of base portions and an average density of the super absorbent polymer particles included in each of the plurality of main groove portion corresponding portions are smaller than an average density of the super absorbent polymer particles included in each of the plurality of base portion corresponding portions is the solution to the problems.

### [EFFECTS OF THE INVENTION]

The absorbent article of the present disclosure is excellent in the diffusibility, the absorbing speed, and the liquid return suppression of the body fluid even when the body fluid is repeatedly absorbed.

### BRIEF DESCRIPTION OF DRAWINGS

FIG. 1 is a plan view of the absorbent article 1 according to the first embodiment.
FIG. 2 is a plan view of the absorbent body 7 of the absorbent article 1 according to the first embodiment.
FIG. 3 is a plan view of the absorbent core 9 of the absorbent article 1 according to the first embodiment.
FIG. 4 is an end surface view of the IV - IV end surface of FIG. 2.
FIG. 5 is an end surface view of the V - V end surface of FIG. 2.
FIG. 6 is an end surface schematic view which shows the flow of the body fluid at the IV - IV end surface of FIG. 2.
FIG. 7 is an outline view of the main groove portion according to an embodiment other than the first embodiment.
FIG. 8 is another outline view of the main groove portion according to another embodiment other than the first embodiment.
FIG. 9 is a view so as to explain the front waist region, the rear waist region, and the crotch portion region of a tape type disposable diaper.
FIG. 10 is a view which shows the outline of the manufacturing apparatus used for the manufacturing method of the absorbent article 1 according to the first embodiment.
FIG. 11 (a) is a main portion enlarged end surface view which schematically shows the state in which the absorbent materials are supplied into the mold member provided in the outer peripheral surface of the suction drum that configures the manufacturing apparatus shown in FIG. 10, and FIG. 11(b) is a main portion enlarged end surface view of the first laminated body which is laminated in the mold member.

### MODE FOR CARRYING OUT THE INVENTION

The present disclosure relates to the following aspects.

### [Aspect 1]

An absorbent article comprising an absorbent core which includes a longitudinal direction, a short length direction, and a thickness direction, and further includes super absorbent polymer particles, wherein
the absorbent core includes a non - skin facing surface and a skin facing surface, and is formed by including a first layer which is present on a non - skin facing surface side and a second layer which is present on a skin facing surface side,
the first layer includes a plurality of groove portions including a plurality of main groove portions which extend along the longitudinal direction and penetrates the first layer in the thickness direction; and a plurality of base portions which extend along the longitudinal direction, and each of the plurality of main groove portions and each of the plurality of base portions extend alternately in the short length direction,
the second layer includes a plurality of main groove portion corresponding portions and a plurality of base portion corresponding portions at positions overlapping with the plurality of main groove portions and the plurality of base portions, respectively, in the thickness direction, and
both an average density of the super absorbent polymer particles included in each of the plurality of base portions and an average density of the super absorbent polymer particles included in each of the plurality of main groove portion corresponding portions are smaller than an average density of the super absorbent polymer particles included in each of the plurality of base portion corresponding portions.

Since the absorbent article according to the present disclosure includes the predetermined plurality of main groove portions and base portions in the first layer, the body fluid which has permeated the plurality of main groove portion corresponding portions of the second layer diffuses in the longitudinal direction, while flowing into the plurality of main groove portions and leaking out to the plurality of base portions. Further, since the absorbent article includes the predetermined average density of the super absorbent polymer particles in each of the plurality of base portions, in each of the plurality of main groove portion corresponding portions, and in each of the plurality of base portion corresponding portions, the swelling of the plurality of base portions and of the plurality of main groove portion corresponding portions which are present in the surroundings of the plurality of main groove portions is suppressed, so as to secure the liquid flow function of the plurality of main groove portions, and further, it is easier for the body fluid which can no longer be retained by the plurality of base portions to be absorbed by the plurality of base portion corresponding portions. In this manner, in the absorbent article, the body fluid is absorbed by a body fluid absorbing cycle in which the body fluid permeates from the plurality of main groove portion corresponding portions to the plurality of main groove portions (hereinbelow, which may also be referred to as "the body fluid permeating action"), the permeated body fluid is drawn from the plurality of main groove portions into the adjacent plurality of base portions (hereinbelow, which may also be referred to as "the body fluid drawing action"), and when the drawn body fluid can no longer be retained by the plurality of base portions, the body fluid is sucked up by the plurality of base portion corresponding portions (hereinbelow, which may also be referred to as "the body fluid sucking up action"). As a result, the absorbent article is excellent in the diffusibility, the absorbing speed, and the liquid return suppression of the body fluid, even when the body fluid is repeatedly absorbed, by repeating the above described body fluid absorbing cycle.

### [Aspect 2]

The absorbent article according to aspect 1, wherein
the average density of the super absorbent polymer particles included in each of the plurality of main groove portion corresponding portions is smaller than the average density of the super absorbent polymer particles included in each of the plurality of base portions.

Since the absorbent article according to the present disclosure includes the predetermined average density of the super absorbent polymer particles in each of the plurality of main groove portion corresponding portions, and in each of the plurality of base portions, the swelling of the plurality of main groove portion corresponding portions is suppressed, so as to make it difficult for the body fluid permeating action to be inhibited. Accordingly, it is easy for the absorbent article to maintain the diffusibility of the body fluid even when the body fluid is repeatedly absorbed.

### [Aspect 3]

The absorbent article according to aspect 1 or 2, wherein
each of the plurality of main groove portions extends, in a plan view, in a zigzag manner or in a corrugated manner, along the longitudinal direction.

Since the absorbent article according to the present disclosure includes the predetermined plurality of main groove portions, the absorbent article demonstrates the operations and effects by the above described aspect 1, and further, since the plurality of main groove portions have the specific shape, it is easy to guide the deformation of the absorbent core, as well as the absorbent article, in the direction in which the plurality of main groove portions extend, and it is easy for the absorbent article to prevent the liquid leakage while fitting with the body of the wearer.

### [Aspect 4]

The absorbent article according to any one of aspects 1 to 3, wherein
each of the plurality of groove portions includes auxiliary groove portions which penetrate the first layer in the thickness direction, and are present in a direction crossing each of the plurality of main groove portions, in a predetermined interval, in a state of being connected to each of the plurality of main groove portions through a base.

The absorbent article according to the present disclosure demonstrates the operations and effects by the above described aspect 1, and further, since each of the plurality of groove portions includes the predetermined auxiliary groove portion, and the auxiliary groove portion has the specific shape, it is easy to guide the deformation of the absorbent core, as well as the absorbent article, in the direction in which the plurality of main groove portions extend, and it is easy for the absorbent article to prevent the liquid leakage while fitting with the body of the wearer.

### [Aspect 5]

The absorbent article according to aspect 4, wherein
the auxiliary groove portions extend in a direction orthogonal to each of the plurality of main groove portions at the base, and each of which includes an end.

The absorbent article according to the present disclosure demonstrates the operations and effects by the above described aspect 1, and further, since the auxiliary groove portion has the specific shape, not only is it difficult for the diffusibility of the body fluid in the longitudinal direction to be inhibited, but also it is easy to guide the deformation of the absorbent core, as well as the absorbent article, in the direction in which the plurality of main groove portions extend, and it is easy for the absorbent article to prevent the liquid leakage while fitting with the body of the wearer.

### [Aspect 6]

The absorbent article according to aspect 4 or 5, wherein
the second layer includes auxiliary groove portion corresponding portions at positions overlapping with the auxiliary groove portions in the thickness direction, and
an average density of the super absorbent polymer particles included in the auxiliary groove portion corresponding portions is smaller than the average density of the super absorbent polymer particles included in each of the plurality of base portion corresponding portions.

Since absorbent article according to the present disclosure includes the predetermined average density of the super absorbent polymer particles in the auxiliary groove portion corresponding portion, and in the plurality of base portion corresponding portions, it is easy for the body fluid to permeate also from the auxiliary groove portion corresponding portion of the second layer to the auxiliary groove portion. Further, when the body fluid reaches the auxiliary groove portion and diffuses in the direction other than the direction along the longitudinal direction, it is easier for the body fluid to leak out to the plurality of base portions also through the auxiliary groove portion so as to be retained, and for the body fluid which can no longer be retained by the plurality of base portions to be absorbed by the plurality of base portion corresponding portions. As a result, the absorbent article according to the present disclosure is excellent in the absorbing speed and the liquid return suppression, even when the body fluid is repeatedly absorbed.

### [Aspect 7]

The absorbent article according to aspect 6, wherein
the average density of the super absorbent polymer particles included in the auxiliary groove portion corresponding portions is smaller than the average density of the super absorbent polymer particles included in each of the plurality of base portions.

Since the absorbent article according to the present disclosure includes the predetermined average density of the super absorbent polymer particles in the auxiliary groove portion corresponding portion, and in each of the plurality of base portions, it is easy for the body fluid to stably permeate from the auxiliary groove portion corresponding portion of the second layer to the auxiliary groove portion.

### [Aspect 8]

The absorbent article according to any one of aspects 5 to 7, wherein
an interval of the auxiliary groove portions adjacent in the short length direction is 10 to 60 % of an interval of the plurality of main groove portions adjacent in the short length direction.

In the absorbent article according to the present disclosure, since the auxiliary groove portions have a predetermined interval, predetermined widths can be secured for the base portions which are present between the auxiliary groove portions, and it is easier for the body fluid which has been supplied to the absorbent core to be absorbed by the base portions, whereby the absorbent article is excellent in the diffusibility, the absorbing speed and the liquid return suppression of the body fluid, even when the body fluid is repeatedly absorbed.

### [Aspect 9]

The absorbent article according to any one of aspects 1 to 8, further comprising a core wrap which is formed by tissue in at least the non - skin facing surface of the absorbent core, wherein
the core wrap is disposed so as to protrude toward the skin facing surface side, in each of the plurality of main groove portions.

Since the absorbent article according to the present disclosure includes the core wrap which is disposed so as to protrude toward the skin facing surface side, in each of the plurality of main groove portions, the diffusibility of the body fluid in the main groove portions can be improved.

Hereinbelow, a preferable embodiment of the absorbent article according to the present disclosure is explained in detail with reference to the drawings. Incidentally, in the present description, unless otherwise noted, "viewing an object (for example, an absorbent article, an absorbent body, an absorbent core, etc.) placed on a horizontal plane in a state of being expanded from an upper side or a lower side in the thickness direction of the object" is simply referred to as "in a plan view". To be more specific, in a case in which the object is an absorbent article, viewing the absorbent article in a state of being expanded from the top sheet side in the thickness direction may be simply referred to as "in a plan view", and in a case in which the object is an absorbent core, viewing the absorbent core in a state in which the absorbent article is expanded from the non - skin facing surface side in the thickness direction may be simply referred to as "in a plan view".

In the present description, various directions, etc., are as follows, unless otherwise noted.

In the present description, "a longitudinal direction" means "the longer length direction of a longitudinal object in a plan view", "a short length direction" means "the shorter length direction of a longitudinal object in a plan view", and "a thickness direction" means "the vertical direction with respect to an object which is placed on a horizontal plane in a state of being expanded". These longitudinal direction, the short length direction, and the thickness direction are in a relationship of mutually being orthogonal to each other.

Further, in the present description, unless otherwise noted, in the thickness direction of an absorbent article, "the relatively closer side with respect to the skin facing surface of the wearer when the absorbent article is being worn" is referred to as "the skin facing surface side", and "the relatively farther side with respect to the skin facing surface of the wearer when the absorbent article is being worn" is referred to as "the non - skin facing surface side". Incidentally, in the present description, "the surface on the skin facing surface side" and "the surface on the non - skin facing surface side" of the various members which configure the absorbent article (for example, the top sheet, the absorbent body, the back sheet, etc.) are respectively simply referred to as "the skin facing surface" and "the non - skin facing surface".

In the present description, "the main groove portion" means not only the groove portion which extends in the direction parallel to the direction of the longitudinal axis line, but also the groove portion which extends along the longitudinal axis line direction. The angle formed by the main groove portion and the longitudinal axis line is preferably less than 45°, more preferably less than 30°, and even more preferably less than 15 °. The main groove portion may extend linearly or nonlinearly, for example in a curved manner.

In the present description, the width of the main groove portion means the length of the main groove portion in the direction orthogonal to the direction in which the main groove portion extends in the target portion.

In the present description, "the auxiliary groove portion" means not only the groove portion which extends in the direction parallel to or substantially parallel to the direction of the short length axis line, but also the groove portion which extends in the direction other than the longitudinal direction. The angle formed by the auxiliary groove portion at the portion communicating with the main groove portion (that is, the base) and the main groove portion (in a case in which the main groove portion extends in a curved manner, the tangent of the main groove portion), is preferably 45 ° or more, more preferably 60° or more, and even more preferably 80° or more. The auxiliary groove portion may extend linearly or nonlinearly, for example in a curved manner.

In the present description, the width of the auxiliary groove portion means the length of the auxiliary groove portion in the direction orthogonal to the direction in which the auxiliary groove portion extends in the target portion.

In the present description, "the front waist region", "the rear waist region" and "the crotch portion region" are technical terms which are used in a case in which the absorbent article is a disposable diaper, and the meanings thereof are as follows.

In a pants type disposable diaper, the front waist region means the region which is sandwiched by a pair of joining portions that join the front panel and the rear panel, among the front panel, and the rear waist region means the region which is sandwiched by the pair of joining portions, among the rear panel.

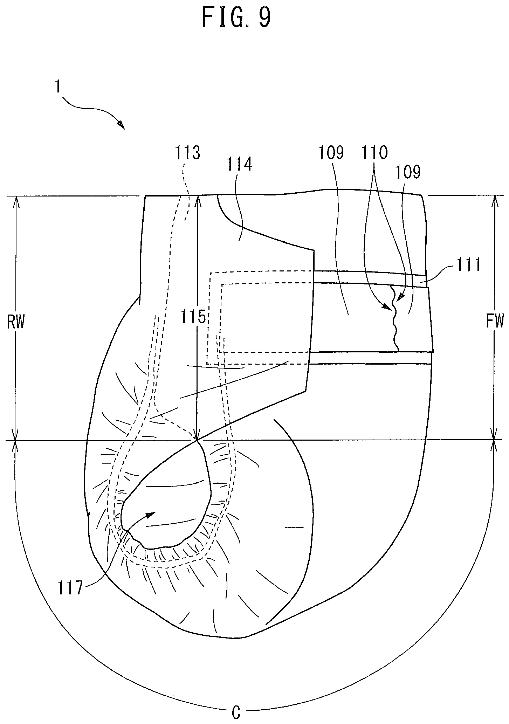

Further, in a pants type disposable diaper, the crotch portion region means the region which is in between the front waist region and the rear waist region. The crotch portion region also corresponds to the region which is sandwiched by a pair of leg opening portions (reference number 117 shown in FIG. 9).

In a tape type disposable diaper, as shown in FIG. 9, the waist regions and the crotch portion region are partitioned in a state in which tips 110 of a pair of tape fasteners 109 are fixed so as to be adjacent to each other in a predetermined tape fastener fixing region 111.

To be more specific, the waist regions (FW + RW) are judged based on a pair of overlapped portions 115 in which the waist forming member 113 of the front panel and the waist forming member 114 of the rear panel overlap with each other, in the absorbent article 1, in a state of being fixed in the above described manner. The front waist region FW means the region in between the pair of overlapped portions 115 among the front panel of the absorbent article 1. In the similar manner, the rear waist region RW means the region in between the pair of overlapped portions 115 among the rear panel of the absorbent article 1. The crotch portion region (C) means the region in between the front waist region FW and the rear waist region RW.

### [Embodiment of the absorbent article]

Hereinbelow, an absorbent article according to the present disclosure is explained in detail.

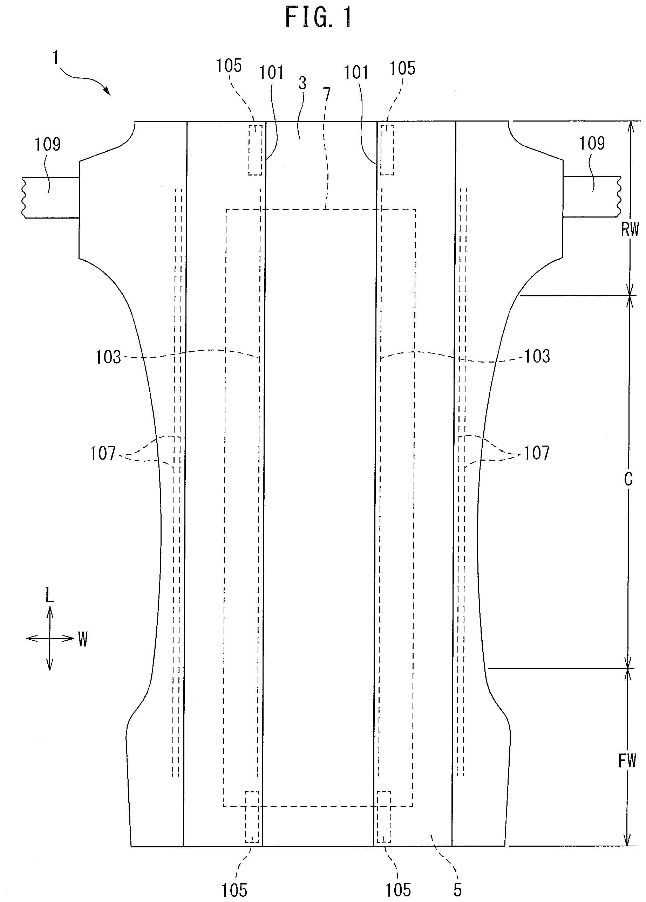

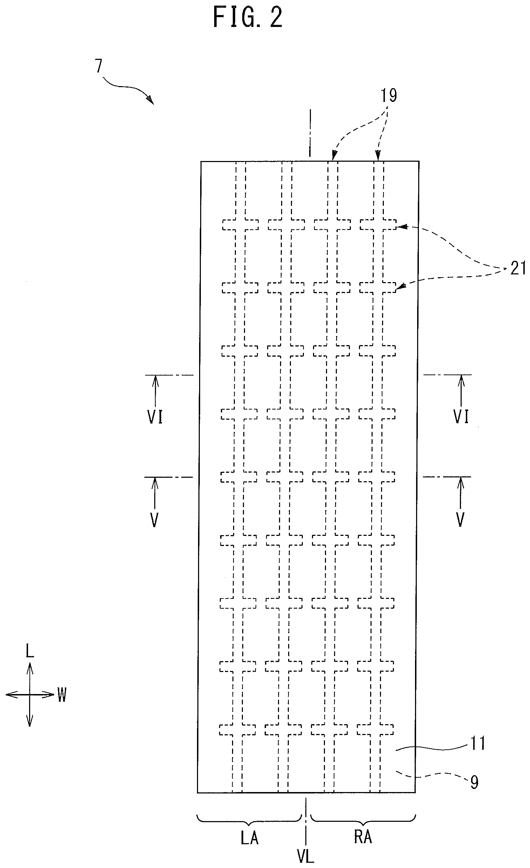

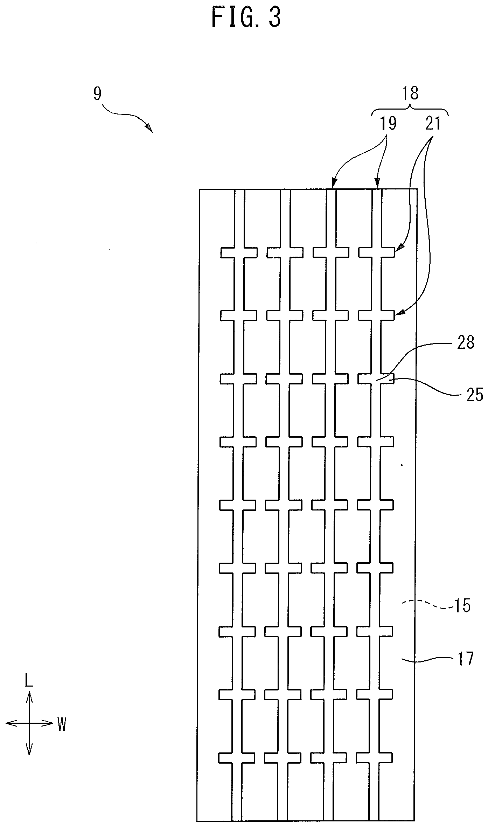

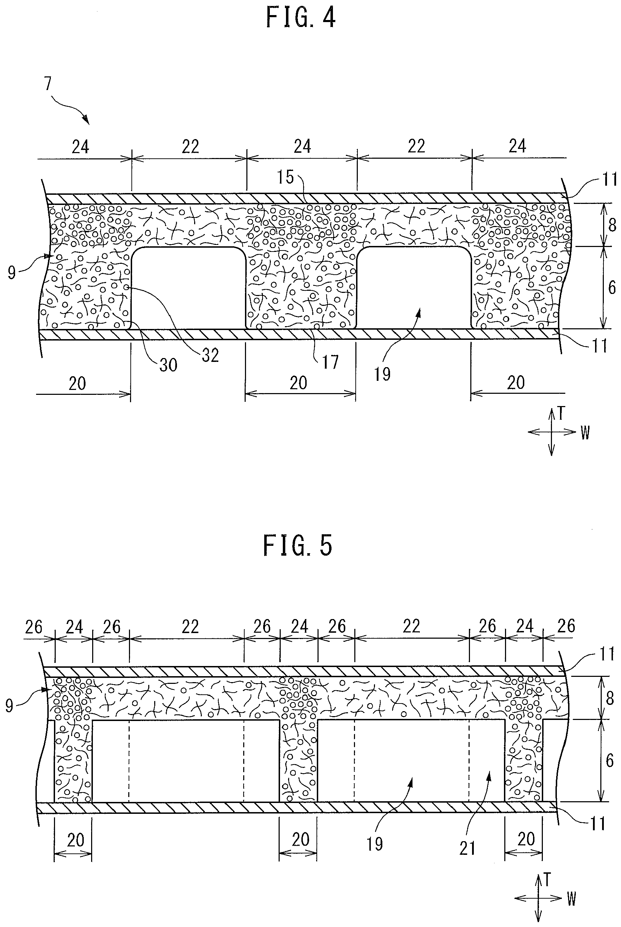

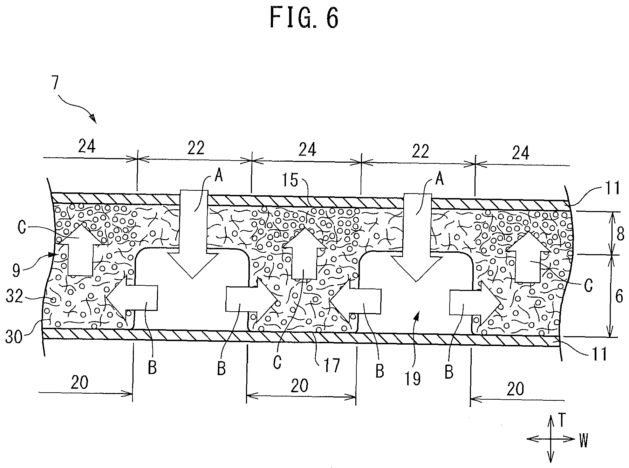

FIG. 1 to FIG. 6 are views so as to explain the absorbent article 1, and more specifically a disposable diaper, according to one embodiment of the present disclosure (the first embodiment). To be more specific, FIG. 1 is a plan view of the absorbent article 1 according to the first embodiment. FIG. 2 is a plan view of the absorbent body 7 of the absorbent article 1 according to the first embodiment. FIG. 3 is a plan view of the absorbent core 9 of the absorbent article 1 according to the first embodiment. FIG. 4 and FIG. 5 are end surface views of the IV - IV end surface and the V - V end surface of FIG. 2, respectively. FIG. 6 is an end surface schematic view which shows the flow of the body fluid at the IV - IV end surface of FIG. 2.

The absorbent article 1 of the first embodiment, as shown in FIG. 1, includes the liquid permeable sheet 3, the liquid impermeable sheet 5, and the absorbent body 7 which is present between the liquid permeable sheet 3 and the liquid impermeable sheet 5.

As shown in FIG. 2 and FIG. 3, the absorbent body 7 includes the longitudinal direction L, the short length direction W, and the thickness direction T, and further includes the absorbent core 9 which has the skin facing surface 15 and the non - skin facing surface 17. Further, in the present embodiment, absorbent core 9 also includes the core wrap 11 formed by a tissue which covers the skin facing surface 15 and the non - skin facing surface 17.

Incidentally, in the first embodiment, the absorbent article 1 includes the same longitudinal direction L and the short length direction W, as the absorbent body 7.

As shown in FIG. 4, the absorbent core 9 includes the non - skin facing surface 17 and the skin facing surface 15, and is formed by including the first layer 6 on the non - skin facing surface side and the second layer 8 on the skin facing surface side.

Further, the first layer 6 includes the plurality of groove portions 18 which include the plurality of main groove portions 19 that extend linearly along the longitudinal direction L and penetrate therethrough in the thickness direction T; and the plurality of base portions 20 which extend along the longitudinal direction L, and the main groove portions 19 and the base portions 20 extend alternately in the short length direction W.

As shown in FIG. 3 and FIG. 4, each of the main groove portions 19 is recessed from the non - skin facing surface 17 in the thickness direction of the absorbent core 9, and extends in the longitudinal direction L.

Further, the second layer 8 includes the plurality of main groove portion corresponding portions 22 at positions overlapping with the plurality of main groove portions 19 in the thickness direction T; and the plurality of base portion corresponding portions 24 at positions overlapping with the plurality of base portions 20 in the thickness direction T.

Further, in the present embodiment, as shown in FIG. 5, the groove portions 18 includes the plurality of auxiliary groove portions 21. Still further, the second layer 8 includes the plurality of auxiliary groove portion corresponding portions 26 at positions overlapping with the plurality of auxiliary groove portions 21 in the thickness direction T.

In the present disclosure, the thickness of each of the first layer and the second layer is preferably 0.1 to 5 mm, more preferably 0.5 to 4 mm, and even more preferably 1 to 3 mm.

Further, in the present disclosure, the thickness of each of the first layer and the second layer may be measured in a non - contact manner as follows by using a laser displacement meter (for example, the high precision two dimensional laser displacement meter LJ - G series (model: LJ - G030) manufactured by Keyence Corporation). The absorbent core which is obtained by blowing a cold spray to the absorbent article so as to peel off the liquid permeable sheet, the liquid impermeable sheet, and the core wrap, is cut into the size of 100 mm × 100 mm, which is regarded as the sample. The sample is placed on a horizontal measuring table so that the non - skin facing surface in which the plurality of groove portions and the base portions are formed faces upward, displacement from the measuring table is measured by the laser displacement meter for five different base portions, and the average value of the five measurement values is regarded as Ax (mm). In the same manner, displacement from the measuring table is measured by the laser displacement meter for five different groove portions (main groove portions), and the average value of the five measurement values is regarded as Ay (mm). The thickness of the second layer is Ay (mm), and the thickness of the first layer is calculated from the difference between Ax (mm) and Ay (mm).

Further, the absorbent core 9 includes the absorbent fibers 30 and the super absorbent polymer particles (SAP) 32, and has a function of absorbing and retaining the body fluid which has been discharged to the absorbent article 1.

In the present disclosure, the average basis weight of the absorbent fibers in each of the first layer and the second layer is preferably 50 to 150 g / m², and more preferably 80 to 100 g / m². Further, the average basis weight of the super absorbent polymer particles in the first layer is preferably 30 to 100 g / m², and more preferably 40 to 60 g / m², and the average basis weight of the super absorbent polymer particles in the second layer is preferably 100 to 180 g / m², and more preferably 120 to 160 g / m². Still further, the ratio of the average basis weight of the super absorbent polymer particles in the first layer with respect to the average basis weight of the super absorbent polymer particles in the entire absorbent core is preferably 20 to 45 %, and more preferably 25 to 35 %.

As shown in FIG. 4, the absorbent core 9 has different average densities of the super absorbent polymer particles in each of the base portions 20, the main groove portion corresponding portions 22, and the base portion corresponding portions 24. To be more specific, the average density of the super absorbent polymer particles in the base portions 20 is smaller than the average density of the super absorbent polymer particles in the base portion corresponding portions 24, and the average density of the super absorbent polymer particles in the main groove portion corresponding portions 22 is smaller than the average density of the super absorbent polymer particles in the base portions 20.

In the present disclosure, the average density of the super absorbent polymer particles in the base portions is preferably 0 to 0.15 g / cm³, and more preferably 0 to 0.1 g / cm³. Further, in the present disclosure, the average density of the super absorbent polymer particles in the main groove portion corresponding portions is preferably 0 to 0.15 g / cm³, and more preferably 0 to 0.1 g / cm³. Still further, in the present disclosure, the average density of the super absorbent polymer particles in the base portion corresponding portions is preferably 0.1 to 0.3 g / cm³, and more preferably 0.1 to 0.2 g / cm³.

Incidentally, in the present description, the measurement method of the average density of the super absorbent polymer particles is as follows. Five samples each having a predetermined length and width are cut out from the absorbent core (for example, 4 mm × 4 mm), the super absorbent polymer particles included in each of the samples are selected, the total mass of the super absorbent polymer particles is measured for each of the samples, the measured values are divided by the volumes of the samples which are obtained by the thickness of the samples (that is described later) and the area of the samples, and the average density can be obtained as the average value of the divided values.

Further, in the present description, in a case of evaluating whether the average density of the super absorbent polymer particles is larger or smaller in each of the base portions, the main groove portion corresponding portions, and the base portion corresponding portions within the absorbent core, the following method may be used. For example, after the absorbent article to be a sample is immersed in a liquid nitrogen and is frozen, the absorbent article is cut in the thickness direction by a razor, and a cross section of the surface crossing the direction in which the main groove portions extend is obtained. Subsequently, the sample is returned to the normal temperature, and a cross sectional image of 50 times magnification is obtained by using an electron microscope (for example, VE7800 manufactured by Keyence Corporation). Further, the cross sectional image, whether the average density of the super absorbent polymer particles is larger or smaller in each of the base portions, the main groove portion corresponding portions, and the base portion corresponding portions is evaluated by visual inspection. Incidentally, in a case of evaluating whether the average density of the super absorbent polymer particles in the auxiliary groove portion corresponding portions to be described later is larger or smaller than the average density of the super absorbent polymer particles in each of the base portions and the base portion corresponding portions, instead of the above described method, by using the cross sectional image of the surface crossing the direction in which the auxiliary groove portions extend, the evaluation can be performed in the similar manner as described above.

As described above, in the present embodiment, both the average density of the super absorbent polymer particles included in each of the base portions 20, and the average density of the super absorbent polymer particles included in each of the main groove portion corresponding portions 22 are smaller than the average density of the super absorbent polymer particles included in each of the base portion corresponding portions 24.

According to the above described configuration, as shown in FIG. 6, in the present embodiment, the body fluid moves within the absorbent core 9 in the following order.
(i) The body fluid which has reached the core wrap 11 permeates the inside of the main groove portion corresponding portions 22 in which the average density of the super absorbent polymer particles is relatively smaller and it is easy for the body fluid to permeate therethrough in the second layer 8, and further, moves to the main groove portions 19 by gravity (as shown in the arrow A).
(ii) The body fluid which has moved to the main groove portions 19, while leaking out to the base portions 20 which are adjacent thereto in the short length direction (as shown in the arrow B), diffuses in the longitudinal direction.
(iii) The body fluid which can no longer be retained by the base portions 20 is sucked up and retained by the base portion corresponding portions 24 in which the average density of the super absorbent polymer particles is the largest within the absorbent core 9 (as shown in the arrow C).

Further, in the present embodiment, since the super absorbent polymer particles within the absorbent core 9 have the above described average density gradient, the swelling of the base portions 20 and the main groove portion corresponding portions 22 which are present in the surroundings of the main groove portions 19 is suppressed, whereby it is easier to maintain the length in the short length direction W and the height in the thickness direction T of the main groove portions 19, and as a result, it is easy to secure the liquid flow function of the main groove portions 19.

In this manner, as shown in FIG. 6, in the absorbent article 1, the body fluid permeates from the main groove portion corresponding portions 22 to the main groove portions 19 as shown in the arrow A by the body fluid permeating action, then, the permeated body fluid is drawn from the main groove portions 19 to the adjacent base portions 20 as shown in the arrow B by the body fluid drawing action, and then when the drawn body fluid can no longer be retained by the base portions 20, the body fluid is sucked up by the base portion corresponding portions 24 as shown in the arrow C by the body fluid sucking up action. In this manner, the absorbent article 1 absorbs the body fluid by the body fluid absorbing cycle which is configured by the body fluid permeating action, the body fluid drawing action, and the body fluid sucking up action. As a result, the absorbent article 1 is excellent in the diffusibility, the absorbing speed, and the liquid return suppression of the body fluid, even when the body fluid is repeatedly absorbed, by repeating the above described body fluid absorbing cycle.

Further, in the present embodiment, as described above, the average density of the super absorbent polymer particles in the main groove portion corresponding portions 22 is smaller than the average density of the super absorbent polymer particles in the base portions 20. According to such a configuration, in the absorbent article 1, since the super absorbent polymer particles within the absorbent core 9 have the above described average density gradient, the swelling of the main groove portion corresponding portions 22 is suppressed, and it is difficult to inhibit the body fluid permeating action. Accordingly, the absorbent article 1 is excellent in maintaining the diffusibility of the body fluid, even when the body fluid is repeatedly absorbed.

Further, as shown in FIG. 3 and FIG. 5, the auxiliary groove portions 21 are recessed from the non - skin facing surface 17 in the thickness direction of the absorbent core 9, communicate with the main groove portions 19, and extend in the short length direction W.

As shown in FIG. 2 to FIG. 4, each of the auxiliary groove portions 21 crosses the plurality of main groove portions 19 and is connected to the main groove portions 19 through the core wrap 11 at the base 28, and the absorbent core 9 is present in the further end of the end 25.

In the present disclosure, when the absorbent core includes the predetermined auxiliary groove portion, in addition to the body fluid permeating action and the body fluid drawing action, since the body fluid leaks out to the base portions also through the auxiliary groove portion and it is easier to be absorbed inside the absorbent core when the body fluid supplied to the absorbent core reaches the auxiliary groove portion and is diffused in the direction other than the longitudinal direction, it is preferable in that the absorbent article is excellent in the absorbing speed and the liquid return suppression, even when the body fluid is repeatedly absorbed. Further, since the auxiliary groove portion has the specific shape, it is easy to guide the deformation of the absorbent core, as well as the absorbent article, in the longitudinal direction, and it is easy for the absorbent article to prevent the liquid leakage while fitting with the body of the wearer.

Further, when the body fluid supplied to the absorbent core reaches the auxiliary groove portion and diffuses in the short length direction, the body fluid leaks out to the base portions through the end of the auxiliary groove portion. At this time, since the auxiliary groove portions are not connected to each other and have an end, it is easy for the body fluid which has been absorbed in the base portions through the end to diffuse in the longitudinal direction, whereby the absorbent article is excellent in the diffusibility, the absorbing speed, and the liquid return suppression, even when the body fluid is repeatedly absorbed.

Further, in the present embodiment, as shown in FIG. 5, the second layer 8 includes the auxiliary groove portion corresponding portions 26 at the position overlapping with the auxiliary groove portions 21 in the thickness direction T.

The average density of the super absorbent polymer particles included in the auxiliary groove portion corresponding portions according to the present disclosure is preferably 0 to 0.15 g / cm³, and more preferably 0 to 0.1 g / cm³.

Further, in the present embodiment, the average density of the super absorbent polymer particles included in the auxiliary groove portion corresponding portions 26 is smaller than the average density of the super absorbent polymer particles included in each of the plurality of base portion corresponding portions 24. According to such a configuration, it is easy for the body fluid to permeate the auxiliary groove portions 21 also from the auxiliary groove portion corresponding portions 26 of the second layer 8. Further, when the body fluid reaches the auxiliary groove portions 21 and diffuses in the direction other than the direction along the longitudinal direction L, it is easier for the body fluid to leak out to the plurality of base portions 20 also through the auxiliary groove portions 21 so as to be retained, and for the body fluid which can no longer be retained by the plurality of base portions 20 to be absorbed by the plurality of base portion corresponding portions 24. As a result, the absorbent article 1 is excellent in the absorbing speed and the liquid return suppression, even when the body fluid is repeatedly absorbed.

Further, in the present embodiment, the average density of the super absorbent polymer particles included in the auxiliary groove portion corresponding portions 26 is smaller than the average density of the super absorbent polymer particles included in each of the plurality of base portions 20. According to such a configuration, it is easy for the body fluid to stably permeate from the auxiliary groove portion corresponding portions 26 of the second layer 8 to the auxiliary groove portions 21.

Further, in the present first embodiment, as shown in FIG. 2, the absorbent body 7 is partitioned into the right side region RA and the left side region LA by the longitudinal direction central axis line VL which bisects the absorbent body 7 in the short length direction W, and the absorbent body 7 includes two main groove portions 19 in each of the right side region RA and the left side region LA.

Further, in the present first embodiment, as shown in FIG. 1, the absorbent article 1 is partitioned into three regions of the front waist region FW, the rear waist region RW and the crotch portion region C between the front waist region FW and the rear waist region RW, in the longitudinal direction L, and the absorbent body 7 is disposed so as to extend over the above described three regions. Further, the absorbent body 7 includes the auxiliary groove portions 21 in each of the three regions.

In the present embodiment, the main groove portions 19 extend linearly along the longitudinal direction L, however, the disposed manner of the groove portions 18 according to the present disclosure is not limited to be above described.

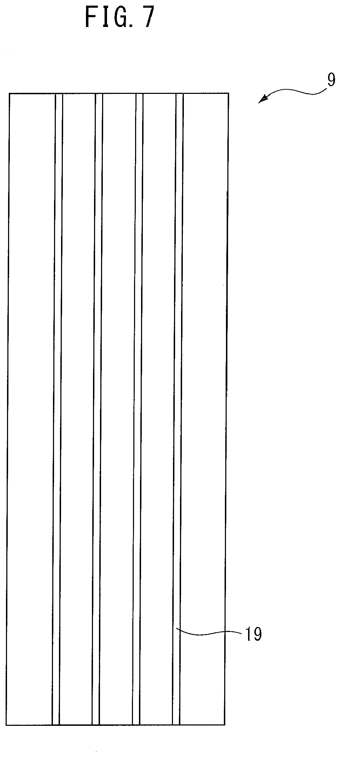

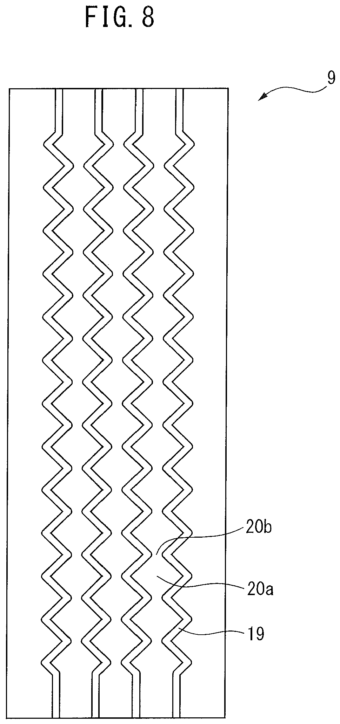

FIG. 7 is an outline view of the main groove portion according to an embodiment other than the first embodiment. Further, FIG. 8 is another outline view of the main groove portion according to another embodiment other than the first embodiment.

As shown in FIG. 7, in another embodiment of the present disclosure, the groove portions 18 are configured only by the main groove portions 19, and the auxiliary groove portions 21 may not be included. Further, in another embodiment of the present disclosure, the main groove portions 19 may not extend over entirely in the longitudinal direction of the absorbent core 9, and the main groove portions 19 may be disposed only in the region of the crotch portion region C in the absorbent article 1.

Further, as shown in FIG. 8, in still another embodiment of the present disclosure, each of the plurality of main groove portions 19 extends, in a plan view, in a zigzag manner along the longitudinal direction L, and is disposed so that the wider base portions 20a in which the length of the width is relatively wider, and the narrower base portions 20b in which the length of the width is relatively narrower, configured by the adjacent main groove portions 19, are alternately formed in the longitudinal direction L.

Further, in still another embodiment of the present disclosure, each of the plurality of main groove portion extends, in a plan view, in a zigzag manner, and may be disposed so that the width of the base portions 20, configured by the adjacent main groove portions 19, is constant, or each of the plurality of main groove portion may extend, in a plan view, in a corrugated manner.

According to such a configuration, since the absorbent article according to the present disclosure includes the predetermined plurality of main groove portions, and further, since the plurality of main groove portions have the specific shape, the rigidity of the absorbent core in the direction in which the plurality of main groove portions extend is decreased, whereby it is easy to guide the deformation of the absorbent core, as well as the absorbent article, in the direction in which the plurality of main groove portions extend, even when the body fluid is repeatedly absorbed, and it is easy for the absorbent article to prevent the liquid leakage while fitting with the body of the wearer.

In the present embodiment, the absorbent core 9 includes the core wrap 11 which is formed by a tissue so as to cover the skin facing surface 15 and the non - skin facing surface 17, and as shown in FIG. 4, in the non - skin facing surface 17, the core wrap 11 is not in contact within the main groove portions 19. However, in the absorbent article according to the present disclosure, the core wrap may be disposed so as to protrude toward the skin facing surface side within the main groove portions in the non - skin facing surface. In such a case, the diffusibility of the body fluid in the main groove portions can be improved.

Further, in the absorbent article according to the present disclosure, the core wrap may be in contact with the main groove portion corresponding portions, or the core wrap may be joined to the main groove portion corresponding portions partially or continuously by compressed portions, etc., in the non - skin facing surface.

In this manner, by the main groove portions being formed so that a portion of the width of the main groove portion corresponding portions are compressed intermittently along the main groove portions, it is more difficult for the wearer to feel the stiffness of the compressed portions. Further, by the main groove portions being formed so that a portion of the width of the main groove portion corresponding portions are compressed continuously in the longitudinal direction, it is easier for the absorbent article to be bent based on the main groove portions, whereby is to be even more improved in the fitting property.

Further, in an absorbent article according to another embodiment of the present disclosure, the core wrap which forms one surface of the absorbent body is joined to the main groove portion corresponding portions by an adhesive agent. According to such a configuration, the similar effect as described above can be obtained.

Further, in the absorbent article according to the present disclosure, in the main groove portions, the core wrap is preferably joined to the entire boundary surface between the main groove portion corresponding portions and the core wrap, that is the bottom portion and the side portion of the main groove portions. According to such a configuration, even after the absorbent article absorbs the body fluid repeatedly, it is even easier for the main groove portions to retain the liquid flow function, and as a result, the absorbent article is to be even more improved in comfortability. Incidentally, the above described side portion means the portion which connects one surface and the bottom portion within the main groove portions.

In the absorbent article according to the present disclosure, the main groove portions have a width of preferably 0.5 to 3.0 times, more preferably 0.8 to 2.5 times, and even more preferably 1.0 to 2.0 times as wide as the thickness of the absorbent body. When the width of the main groove portions is within the above described range, it is easier for the main groove portions to retain the liquid flow function after the absorbent article absorbs the body fluid.

In the present description, unless otherwise noted, the thickness (mm) of a target (for example, an absorbent body, or an absorbent core) is measured in the following manner.

FS - 60DS [the measurement surface of 44 mm (in diameter), the measurement pressure of 3 g / cm²] manufactured by Daiei Kagaku Seiki MFG. Co., Ltd. is prepared, five different portions of the object are applied with pressure under the standard condition (the temperature of 23 ± 2 °C, the relative humidity of 50 ± 5 %), the thickness of each of the portions ten seconds after the pressure is applied is measured, and the average value of the five measurement values is regarded as the thickness of the absorbent body.

In the absorbent article according to the present disclosure, the interval of the auxiliary groove portions adjacent in the short length direction is preferably 10 to 60 %, and more preferably 15 to 45 % of the interval of the plurality of main groove portions adjacent in the short length direction. When the auxiliary groove portions have the predetermined interval in this manner, a predetermined width can be secured for the base portions which are present in between the auxiliary groove portions, whereby it is easier for the body fluid absorbed by the base portions through the auxiliary groove portions to diffuse in the longitudinal direction, and the absorbent article is excellent in the diffusibility, the absorbing speed, and the liquid return suppression, even when the body fluid is repeatedly absorbed.

In the first embodiment, the absorbent body 7 includes the main groove portions 19 in both of the right side region RA and the left side region LA, in the short length direction W. According to such a configuration, it is easier to let the body fluid 31 which has reached the absorbent body 7 diffuse in a wide range of the absorbent body 7 and be absorbed in the wide range of the absorbent body 7, whereby it is difficult for the absorbed body fluid to return from the surface of the liquid permeable sheet 3.

In the first embodiment, the absorbent body 7 includes the auxiliary groove portions 21 in the three regions of the front waist region FW, the rear waist region RW, and the crotch portion region C. According to such a configuration, the absorbent article 1 is excellent in the fitting property in the longitudinal direction. Further, since the body fluid which has diffused in the longitudinal direction L by passing through the main groove portions 19 diffuses in the short length direction W from the end 25 by passing through the auxiliary groove portions 21, and is absorbed in a wide range of the absorbent body 7, the absorbent article 1 is excellent in the liquid return suppression. Further, even after the auxiliary groove portions 21 are narrowed or are almost disappeared according to the amount of the body fluid, by the body fluid absorbed in the short length direction W, when the body fluid 31 further reaches the absorbent body 7, while the body fluid 31 passes through the bases of the narrowed or almost disappeared auxiliary groove portions 21, the body fluid 31 is further diffused in the longitudinal direction L of the absorbent body 7 by passing through the main groove portions 19, diffuses in the short length direction W from the end 25 by a capillary phenomenon by passing through the auxiliary groove portions 21 which are positioned on further end portion in the longitudinal direction L of the absorbent body 7, whereby the body fluid 31 is absorbed in a wide range of the absorbent body 7. As a result, since the absorbent article 1 is excellent in the liquid return suppression, the absorbent article 1 is excellent in the comfortability.

In the first embodiment, the core wrap 11 is not joined to the auxiliary groove portion corresponding portions 26. According to such a configuration, even after the auxiliary groove portions 21 are narrowed or are almost disappeared according to the amount of the body fluid, by the body fluid absorbed in the short length direction W, when the body fluid 31 further reaches the absorbent body 7, while the body fluid passes through the bases of the narrowed or almost disappeared auxiliary groove portions 21, the body fluid is further diffused in the longitudinal direction L of the absorbent body 7 by passing through the main groove portions 19, diffuses in the short length direction W and in the longitudinal direction L from the end 25 by passing through the auxiliary groove portions 21 which are positioned on further end portion in the longitudinal direction L of the absorbent body 7, whereby the body fluid is absorbed in a wide range of the absorbent body 7. As a result, since the absorbent article 1 is excellent in the liquid return suppression, the absorbent article 1 is excellent in the comfortability.

In this manner, in the absorbent article according to the present disclosure, preferably, the auxiliary groove portions are connected to the main groove portions at the bases, and the absorbent core is present in the further end of such ends, and the auxiliary groove portions are present with preferably 0.05 to 1 auxiliary groove portion / cm per unit length of the main groove portion, and more preferably 0.1 to 0.5 auxiliary groove portion / cm per unit length of the main groove portion. In this manner, when the auxiliary groove portions are provided so that the number of the auxiliary groove portions are within the predetermined range, even when the absorbent article absorbs the body fluid repeatedly, the absorbent article has the fitting property in the direction in which the main groove portions extend, and further, when the body fluid supplied to the absorbent body reaches the auxiliary groove portions and is diffused in the direction other than the longitudinal direction, it is easier for the body fluid to be absorbed efficiently in the base portions by the capillary phenomenon through the end of the partitioned auxiliary groove portions, whereby it is easier for the liquid return suppression of the absorbent body to be improved.

The width of the auxiliary groove portions is not particularly limited, however, when the width of the auxiliary groove portions is set as Ws, the total length of a main groove portion is set as Lp, and the number of the auxiliary groove portions provided on one side of one main groove portion is set as n, the auxiliary groove portions preferably satisfy 0.05 Lp / n ≤ Ws ≤0.50 Lp / n. When the auxiliary groove portions are provided so that the width thereof is within the predetermined range, in a case in which the body fluid which has been supplied to the absorbent body reaches the auxiliary groove portions and is diffused in the direction other than the longitudinal direction, it is easier for the body fluid to be absorbed efficiently in the base portions by the capillary phenomenon through the end of the partitioned auxiliary groove portions, whereby it is easier for the absorbency (especially, the absorbing speed), and the liquid return suppression of the absorbent body to be improved.

Further, in the absorbent article according to the present disclosure, the ends of the auxiliary groove portions are present with preferably 0.05 to 1 end / cm², and more preferably 0.1 to 0.5 end / cm² per unit area of the absorbent body. In this manner, when the auxiliary groove portions are provided so that the ends thereof are within the predetermined range, it is easier for the liquid return suppression of the absorbent body to be improved by the similar reasons as described above.

Incidentally, in the first embodiment, as shown in FIG. 1, the absorbent article 1 includes a pair of leakage barriers 101 including elastic members 103, the fixed potions 105 so as to fix the leakage barriers 101 to the liquid permeable sheet 3, the elastic members 107, the tape fastener 109, etc., however, since these members are known in this technical field, the explanations thereof are omitted.

### [Manufacturing method of the absorbent article]

In a case in which the absorbent article 1 which has the above described configuration is manufactured, although the manufacturing method is not particularly limited, the following method may be used, for example.

Incidentally, in the present description, "the conveying direction of a material or a product" is referred to as "the MD direction", "the direction orthogonal to the MD direction on a horizontal plane" (in other words, the width direction of the manufacturing line) is referred to as "the CD direction", and "the direction orthogonal to the MD direction and the CD direction" (in other words, the vertical direction of the manufacturing line) is referred to as "the TD direction".

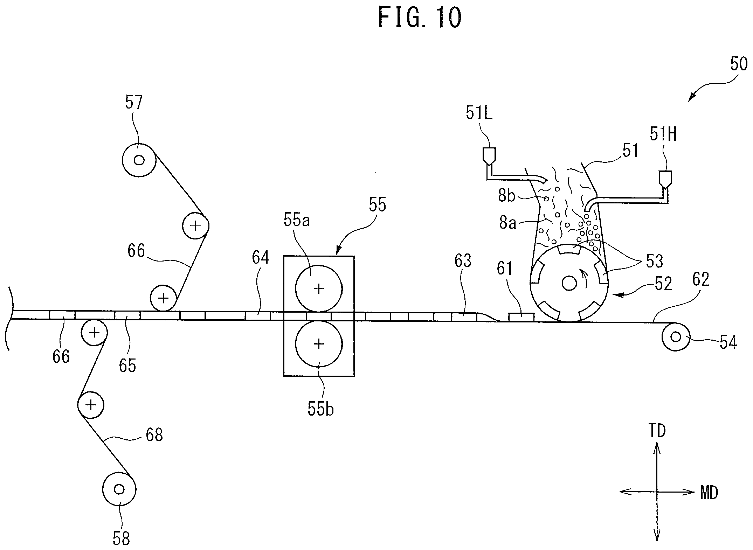

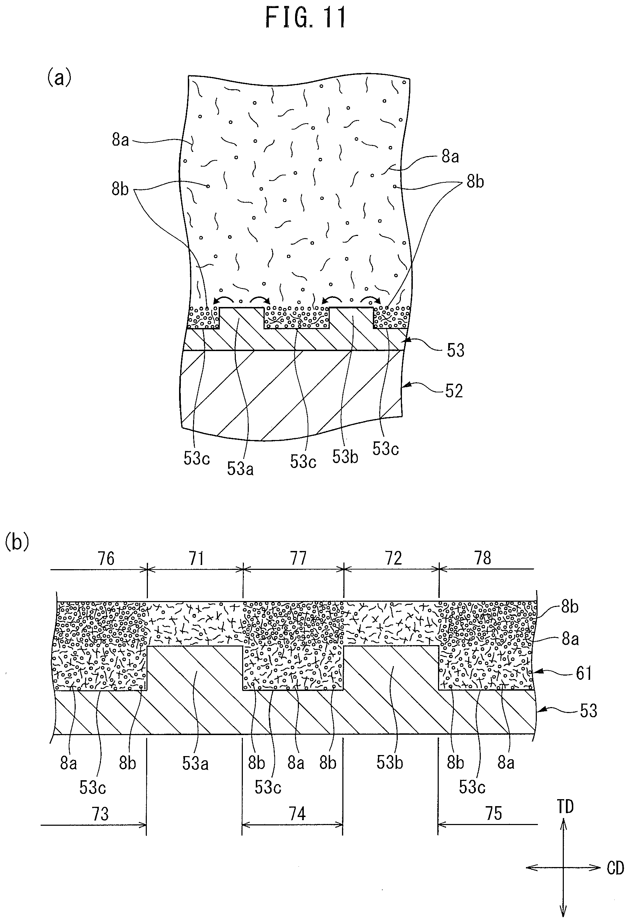

FIG. 10 shows one example of the manufacturing apparatus so as to manufacture the absorbent article 1 according to the first embodiment. Further, FIG. 11 (a) is a main portion enlarged end surface view which schematically shows the state in which the absorbent materials are supplied into the mold member 53 provided in the outer peripheral surface of the suction drum 52 that configures the manufacturing apparatus shown in FIG. 10, and FIG. 11(b) is a main portion enlarged end surface view of the first laminated body 61 which is laminated in the mold member 53.

The manufacturing apparatus 50 includes the conveying pipe 51 which conveys the absorbent materials including the opened water absorbent fibers 8a, and the super absorbent polymer particles 8b; and the freely rotatable suction drum 52 which sucks the absorbent materials inside the conveying pipe 51 so as to let the absorbent materials laminate within the plurality of recessed mold members 53 which are arranged at regular intervals along the circumferential direction in the outer peripheral surface, whereby forms the first laminated body 61 which is to be the absorbent core 9 of the absorbent body 7 in the later step.

Further, the conveying pipe 51 includes the conveying pipe nozzle 51L which discharges the super absorbent polymer particles 8b in a relatively smaller amount, and the conveying pipe nozzle 51H which discharges the super absorbent polymer particles 8b in a relatively larger amount. Incidentally, in the present embodiment, the mass ratio of the super absorbent polymer particles included in each of the conveying pipe nozzle 51L and the conveying pipe nozzle 51H is 30 : 70.

Further, the manufacturing apparatus 50 includes the unwinding roll for core wrap continuous body 54 which lets the first laminated body 61 on the outer peripheral surface of the suction drum 52 be placed thereon, winds out the long core wrap continuous body 62 which covers the first laminated body 61, and covers the first laminated body 61 with the core wrap continuous body 62, whereby forms the second laminated body 63.

Further, the manufacturing apparatus 50 includes the pressing device 55 including the vertical (the TD direction) pair of pressing rolls 55a, 55b which apply pressure and compress the second laminated body 63 in the thickness direction.

Further, the manufacturing apparatus 50 includes, in the further downstream from the pressing device 55 in the conveying direction MD, the unwinding roll for liquid permeable sheet continuous body 57 which winds out and laminates the long liquid permeable sheet continuous body 66 which is to be the liquid permeable sheet 3, for one surface (the upper surface in the case of FIG. 10) of the third laminated body 64 which has been subjected to the pressing treatment.

Further, the manufacturing apparatus 50 includes the unwinding roll for liquid impermeable sheet continuous body 58 which winds out and joins the long liquid impermeable sheet continuous body 68 which is to be the liquid permeable sheet 3, for the opposite surface (the lower surface in the case of FIG. 10) to the liquid permeable sheet continuous body 66 in the fourth laminated body 65 which is formed by laminating the liquid permeable sheet continuous body 66 on the third laminated body 64, whereby forms the fifth laminated body 67.

Incidentally, in the further downstream from the unwinding roll for liquid impermeable sheet continuous body 58 in the conveying direction MD, the manufacturing apparatus 50 further includes a cutting device (which is not shown) which cuts the laminated body 67 into the shape of the absorbent article 1 as a product so as to obtain the absorbent article 1 as a single body, and each device (which is not shown) which crimps the leakage barriers 101 and the tape fastener 109 onto the fifth laminated body 67, however, since these are normal devices known in the technical field, the detailed explanations thereof are omitted.

In a case of manufacturing the absorbent article 1 by using the above described manufacturing apparatus 50, basically, the first step of forming the first laminated body 61, the second step of forming the second laminated body 63 by covering the first laminated body 61 with the core wrap continuous body 62, and the third step of compressing the second laminated body 63 by the pressing device 55 in the thickness direction so as to obtain the third laminated body 64 are sequentially performed. Further, the fourth step of laminating the liquid permeable sheet continuous body 66 on the third laminated body 64 so as to obtain the fourth laminated body 65, and the fifth step of joining the liquid impermeable sheet continuous body 68 to the fourth laminated body 65 so as to obtain the fifth laminated body 67 are sequentially performed.

First, the first step of forming the first laminated body 61 which in the end is to configure the absorbent core 9 of the absorbent body 7 is performed.

The first step sucks the absorbent materials which includes the water absorbent fibers 8a and the super absorbent polymer particles 8b by the suction drum 52 through the conveying pipe 51, lets the absorbent materials be laminated within the mold members 53 in the outer peripheral surface of the suction drum 52, whereby forms the first laminated body 61.

At this time, as shown in FIG. 11 (a), the absorbent materials are supplied from above to the inside of the mold members 53, and each of the mold members 53 includes four pairs of protruded portions (only 53a, 53b are shown in FIG. 11) with a cross section rectangular shape which extend in the circumferential direction of the suction drum 52, in the bottom portion thereof. To be more specific, each of the protruded portions 53a, 53b has a rectangular shape. The protruded portions 53a, 53b are arranged at positions corresponding to the positions of the main groove portions 19 of the absorbent body 7, so as to have the shape in the length direction and the width which correspond to the shape in the length direction and the width of the main groove portions.

### [The first layer laminating step]

First, when the absorbent materials are supplied within the mold members 53 from the conveying pipe nozzle 51L which includes the super absorbent polymer particles 8b with a relatively smaller discharging amount, since the water absorbent fibers 8a are light and soft, it is difficult for the water absorbent fibers 8a to be bounced back even when collided with the pair of protruded portions 53a, 53b, whereby is accumulated approximately evenly inside the molding members 53.

On the other hand, the super absorbent polymer particles 8b, at the stage when the absorbent materials begin to be supplied to the inside of the molding members 53, collide with the tip surface of the protruded portions 53a, 53b, bounce back and move without staying at the collided position, and finally reach the portion other than the protruded portions 53a, 53b in the bottom portion 53c to be accumulated. This is because, in addition to the fact that the super absorbent polymer particles 8b are normally granular, the mass per one super absorbent polymer particle is relatively large, and it is easy for the super absorbent polymer particles 8b to be influenced by their own kinetic energy also due to the suction of the suction drum 52, whereby it is easy for the super absorbent polymer particles 8b to be bounced back when collided with the tip surface of the protruded portions 53a, 53b, and is difficult for the super absorbent polymer particles 8b to stay at the collided position. As described above, the first layer 6 in the absorbent core 9 is to be laminated.

### [The second layer laminating step]

Subsequently, instead of the conveying pipe nozzle 51L, the absorbent materials are supplied from the conveying pipe nozzle 51H which includes the super absorbent polymer particles 8b with a relatively larger discharging amount and is present at a lower position than the conveying pipe nozzle 51L. In the mold members 53, although the water absorbent fibers 8a are accumulated also on the protruded portions 53a, 53b, since the super absorbent polymer particles 8b are included with a relatively larger discharging amount, when the super absorbent polymer particles 8b collide therewith, it is still difficult for the impact to be absorbed even by the softness of the water absorbent fibers 8a, and it is easy for the super absorbent polymer particles 8b to be bounced back. As a result, in the end, as shown in FIG. 11 (b), in the first laminated body 61, a plurality of portions are to be present in which the average density of the super absorbent polymer particles 8b with respect to the absorbent core 9 is different in the width direction. As described above, the second layer 8 in the absorbent core 9 is to be laminated.

In other words, at the portions corresponding to just above the protruded portions 53a, 53b inside the molding member 53, the average density of the super absorbent polymer particles is the smallest by the movement of the super absorbent polymer particles 8b due to the collision with the protruded portions 53a, 53b, whereby are the small average density regions 71, 72. Further, at the portions along the side wall portions of the protruded portions 53a, 53b and the vicinity thereof, since the super absorbent polymer particles 8b which have moved thereto are present, the average density of the super absorbent polymer particles is relatively large, whereby are the medium average density regions 73, 74, 75. Still further, at the portions which is largely influenced by the super absorbent polymer particles 8b being bounced back from the protruded portions 53a, 53b inside the molding member 53, the average density of the super absorbent polymer particles is the largest, whereby are the large average density regions 76, 77, 78. Further, the small average density regions correspond to the main groove portion corresponding portions 22, the medium average density regions correspond to the base portions 20, and the large average density regions correspond to the base portion corresponding portions 24, respectively.

After the first step, the first laminated body 61 within the mold members 53 is placed on the core wrap continuous body 62 with an adhesive agent such as a hot melt type adhesive agent, etc., being applied therebetween, by the rotation of the suction drum 52, the outer peripheral surface of the first laminated body 61 is covered by the core wrap continuous body 62 so as to obtain the second laminated body 63, whereby the second step is performed.

In the second step, first, the rotating suction drum 52 transfers and places the first laminated body 61 within the mold members 53 on the core wrap continuous body 62 which is wound out from the unwinding roll for core wrap continuous body 54 and moves in the conveying direction MD. Then, by the bending means which is not shown, the core wrap continuous body 62 is bent in the width direction CD orthogonal to the conveying direction MD along the outer peripheral surface of the first laminated body 61, the core wrap continuous body 62 is covered by being wound to the first laminated body 61, whereby the long second laminated body 63 is obtained.

After the second step, the third step of compressing the second laminated body 63 in the thickness direction so as to obtain the third laminated body 64 is performed. In the third step, by letting the second laminated body 63 pass through the pair of pressing rolls 55a, 55b of the pressing device 55, the second laminated body 63 shown in FIG. 11 (a) is compressed in the thickness direction TD. At this time, the third laminated body 64 is formed.

After the third step is terminated, on the upper surface of the third laminated body 64, the liquid permeable sheet continuous body 66 which is wound out from the unwinding roll for liquid permeable sheet continuous body 57 is laminated, with an adhesive agent such as a hot melt type adhesive agent, etc., being applied therebetween, so as to obtain the long fourth laminated body 65, whereby the fourth step is performed.

Further, after the fourth step is terminated, to the lower surface of the fourth laminated body 65, the liquid impermeable sheet continuous body 68 which is wound out from the unwinding roll for liquid impermeable sheet continuous body 58 is joined, with an adhesive agent such as a hot melt type adhesive agent, etc., being applied therebetween, so as to obtain the long fifth laminated body 67, whereby the fifth step is performed.

After the fifth step is terminated, the fifth laminated body 67 is cut into the shape of the absorbent article 1 by a cutting device.

As a result, the absorbent article 1 is completed.

Incidentally, in the above described manufacturing method, the aspect of the mold members including the protruded portions for forming the main groove portions is explained, however, in a manufacturing method of the absorbent article according to the present disclosure, the mold members may include protruded portions for forming the auxiliary groove portions.

Further, each step may be suitably modified, unless deviating from the purpose of the present disclosure.

### [Examples]

Hereinbelow, the present disclosure is explained by mentioning examples, however, the present disclosure is not limited to these examples.

### [Manufacturing Example 1]

The absorbent core No. 1 which included pulp (with an average basis weight : 170 g / m²) and super absorbent polymer particles (with an average basis weight: 200 g / m²), and had a size of 350 mm × 120 mm (the longitudinal direction × the short length direction) was manufactured according to the above described manufacturing method. In the absorbent core No. 1, four main groove portions (with each width : 4 mm) were disposed with an interval of 18 mm in the first layer. Further, the average basis weight ratio of the super absorbent polymer particles in the first layer and the second layer was 30 : 70. Further, when the cross sectional image of the surface crossing the direction in which the main groove portions extend in the absorbent core No. 1 was visually evaluated, it was confirmed that both the average density of the super absorbent polymer particles included in the base portions and the average density of the super absorbent polymer particles included in the main groove portion corresponding portions were smaller than the average density of the super absorbent polymer particles included in the base portion corresponding portions (hereinbelow, such an evaluation result is referred to as "satisfying the SAP gradient condition").

Incidentally, the thickness of the absorbent core was 2.0 mm.

The absorbent core No. 1 was covered by two pieces of tissue as the core wraps (with each basis weight : 16 g / m², and each size of 400 mm × 150 mm) with a hot melt adhesive agent applied therebetween. The absorbent core No. 1 which was covered by two pieces of tissue was pressed by a hydraulic pressing machine so as to adjust the thickness of the absorbent core, whereby the absorbent body No. 1 was formed.

A liquid permeable sheet (an air through nonwoven fabric) was adhered onto the second layer side of the absorbent body No. 1, and a liquid impermeable sheet (a polyethylene film) was adhered onto the first layer side, whereby a simple absorbent article No. 1 was manufactured.

### [Manufacturing Example 2]

The absorbent article No. 2 was manufactured in the same manner as the manufacturing example 1 except for further disposing 14 auxiliary groove portions (with each width of 4 mm) for each of the four main groove portions, with an interval of 20 mm and the length of 6 mm symmetrically in the first layer of the absorbent core No. 1. Incidentally, when the cross sectional image of the surface crossing the direction in which the main groove portions extend in the absorbent core sample No. 2 in the absorbent article No. 2 was visually evaluated, the above described SAP gradient condition was satisfied.

### [Manufacturing Example 3]

The absorbent article No. 3 was manufactured in the same manner as the manufacturing example 1 except for making each of the main groove portions of the absorbent core No. 1 into a zigzag shape with a longitudinal direction pitch of 24 mm and a short length direction protruded length of 12 mm. Incidentally, when the cross sectional image of the surface crossing the direction in which the main groove portions extend in the absorbent core sample No. 3 in the absorbent article No. 3 was visually evaluated, the above described SAP gradient condition was satisfied.

### [Manufacturing Example 4]

The absorbent article No. 4 was manufactured in the same manner as the manufacturing example 1 except for making the basis weight ratio of the super absorbent polymer particles in the first layer and the second layer in the absorbent core No. 1 be 50 : 50, and supplying the pulp and the super absorbent polymers in a mixed manner to the mold members. Incidentally, when the cross sectional image of the surface crossing the direction in which the main groove portions extend in the absorbent core sample No. 4 in the absorbent article No. 4 was visually evaluated, the above described SAP gradient condition was not satisfied.

### [Manufacturing Example 5]

The absorbent article No. 5 was manufactured in the same manner as the manufacturing example 2 except for making the basis weight ratio of the super absorbent polymer particles in the first layer and the second layer in the absorbent core No. 2 be 50 : 50, supplying the pulp and the super absorbent polymers in a mixed manner to the mold members, and connecting the auxiliary groove portions adjacent in the short length direction with each other. Incidentally, when the cross sectional image of the surface crossing the direction in which the main groove portions extend in the absorbent core sample No. 5 in the absorbent article No. 5 was visually evaluated, the above described SAP gradient condition was not satisfied.

### [Examples 1 to 3, and Comparative examples 1 and 2]

The absorbent articles No. 1 to No. 5 were subjected to the absorbency test as defined below, and the absorbing speed, the liquid return amount, and the diffusion length on the liquid permeable sheet side were evaluated. The results are shown in Table 1.

### [Absorbency test]

(1) The disposable articles are set to a U-shaped instrument the side view of which is substantially U-shaped. Incidentally, the absorbent article is set so that the central position of the absorbent body in the longitudinal direction matches the central portion of the U-shaped instrument (the position at which the height is the lowest).

### < The first cycle >

(2) 80 mL of artificial urine (the first time) is injected from a burette with the speed of 80 mL / 10 sec to the central position of the absorbent body.
(3) The time from the start of injection of the artificial urine of the first time until the artificial urine within the U-shaped instrument disappears is recorded as the absorbing time (80 mL).
(4) After 3 minutes from the start of injection of the artificial urine of the first time, the outline of the region in which the artificial urine has diffused (80 mL) in the liquid permeable sheet of the absorbent articles is recorded.

### < The second cycle >

(5) After 10 minutes from the start of injection of the artificial urine of the first time, 80 mL of artificial urine (the second time) is injected from a burette with the speed of 80 mL / 10 sec to the central position of the absorbent body.
(6) The time from the start of injection of the artificial urine of the second time until the artificial urine within the U-shaped instrument disappears is recorded as the absorbing time (160 mL).
(7) After 3 minutes from the start of injection of the artificial urine of the second time, the outline of the region in which the artificial urine has diffused (160 mL) in the liquid permeable sheet of the absorbent articles is recorded.

### < The third cycle >

(8) The operations of (5) to (7) are repeated, the absorbing time (240 mL) is measured, and the outline (240 mL) is recorded.

### < The fourth cycle >

(9) The operations of (5) to (7) are repeated, the absorbing time (320 mL) is measured, and the outline (320 mL) is recorded.
(10) After 4 minutes from the start of injection of the artificial urine of the fourth time, the absorbent articles are removed from the U-shaped instrument, the absorbent articles are expanded on an acrylic flat plate so that the liquid permeable sheet is to be the upper surface, and is left still for one minute.
(11) After 5 minutes from the start of injection of the artificial urine of the fourth time, approximately 60 g of filter paper with the size of 100 mm × 100 mm is placed still on the liquid permeable sheet of the absorbent articles with the artificial urine injection point as the center. Further, 3.5 kg of weight with the size of 100 mm × 100 mm × 50 mm (height) is placed still thereon. Incidentally, as for the filter paper, the mass before the test is measured in advance.
(12) After 8 minutes from the start of injection of the artificial urine of the fourth time, the weight is removed, the mass of the filter paper is measured, the mass of the filter paper before the test is subtracted, and the difference is regarded as the liquid return amount.
(13) From the outlines of the region in which the artificial urine has diffused (160 mL to 320 mL), the diffusion length of the artificial urine in the longitudinal direction of the absorbent article is measured.

Incidentally, the artificial urine is prepared by dissolving 200 g of urea, 80 g of sodium chloride, 8 g of magnesium sulfate, 3g of calcium chloride, and approximately 1 g of a dye : blue No. 1, in 10 L of ion exchanged water.

**[Table 1]**

| Example No. | | Example 1 | Example 2 | Example 3 | Comparative Example 1 | Comparative Example 2 |
|---|---|---|---|---|---|---|
| Absorbent article No. | | No. 1 | No. 2 | No. 3 | No. 4 | No. 5 |
| SAP gradient condition | | Satisfied | Satisfied | Satisfied | Not satisfied | Not satisfied |
| Main groove portions | | Present | Present | Present (zigzag) | Present | Present |
| Auxiliary groove portions | | Not present | Present | Not present | Not present | Present (connected) |
| Absorbing time / sec | 80 mL | 19 | 17 | 18 | 19 | 18 |
| | 160 mL | 18 | 18 | 19 | 20 | 19 |
| | 240 mL | 30 | 29 | 32 | 35 | 38 |
| | 320 mL | 78 | 78 | 79 | 97 | 120 |
| Liquid return amount / g | 320 mL | 69 | 68 | 78 | 82 | 91 |
| Diffusion length / mm | 80 mL | 180 | 175 | 168 | 185 | 139 |
| | 160 mL | 182 | 177 | 172 | 185 | 142 |
| | 240 mL | 218 | 205 | 216 | 211 | 186 |
| | 320 mL | 253 | 245 | 251 | 239 | 195 |

When the absorbent articles No. 1 to No. 3 were compared with the absorbent articles No. 4 and No. 5, which had been subjected to the absorbency test of four cycles, it was confirmed that the absorbent articles No. 1 to No. 3 were better in either of the absorbing time, the liquid amount, and the diffusion length.

### [Comfortability test]

Next, when the absorbent articles No. 1 to No. 5 were worn by a plurality of volunteer subjects, answers were received that in the absorbent articles No. 1 to No. 3, the absorbing property was good and there was little discomfort even after four times or more of urinations. Further, answers were received that the rigidity is relatively low and the fitting property is excellent even after four times or more of urinations in the absorbent articles No. 2 and No. 3. In this manner, it was understood that absorbent articles No. 2 and No. 3 demonstrate efficient absorbing property and are excellent in the fitting property even after the body fluid is repeatedly absorbed.

### REFERENCES SIGNS LIST

- 1: absorbent article
- 3: liquid permeable sheet
- 5: liquid impermeable sheet
- 7: absorbent body
- 9: absorbent core
- 11: core wrap
- 13: diffusion sheet
- 15: skin facing surface
- 17: non - skin facing surface
- 18: groove portion
- 19: main groove portion
- 21: auxiliary groove portion
- 25: end
- 28: base
- L: longitudinal direction
- W: short length direction
- RA: right side region
- LA: left side region

## Claims

1. An absorbent article comprising an absorbent core which includes a longitudinal direction, a short length direction, and a thickness direction, and further includes super absorbent polymer particles, wherein
the absorbent core includes a non - skin facing surface and a skin facing surface, and is formed by including a first layer which is present on a non - skin facing surface side and a second layer which is present on a skin facing surface side,
the first layer includes a plurality of groove portions including a plurality of main groove portions which extend along the longitudinal direction and penetrates the first layer in the thickness direction; and a plurality of base portions which extend along the longitudinal direction, and each of the plurality of main groove portions and each of the plurality of base portions extend alternately in the short length direction,
the second layer includes a plurality of main groove portion corresponding portions and a plurality of base portion corresponding portions at positions overlapping with the plurality of main groove portions and the plurality of base portions, respectively, in the thickness direction, and
both an average density of the super absorbent polymer particles included in each of the plurality of base portions and an average density of the super absorbent polymer particles included in each of the plurality of main groove portion corresponding portions are smaller than an average density of the super absorbent polymer particles included in each of the plurality of base portion corresponding portions.

2. The absorbent article according to claim 1, wherein
the average density of the super absorbent polymer particles included in each of the plurality of main groove portion corresponding portions is smaller than the average density of the super absorbent polymer particles included in each of the plurality of base portions.

3. The absorbent article according to claim 1 or 2, wherein
each of the plurality of main groove portions extends, in a plan view, in a zigzag manner or in a corrugated manner, along the longitudinal direction.

4. The absorbent article according to any one of claims 1 to 3, wherein
each of the plurality of groove portions includes auxiliary groove portions which penetrate the first layer in the thickness direction, and are present in a direction crossing each of the plurality of main groove portions, in a predetermined interval, in a state of being connected to each of the plurality of main groove portions through a base.

5. The absorbent article according to claim 4, wherein
the auxiliary groove portions extend in a direction orthogonal to each of the plurality of main groove portions at the base, and each of which includes an end.

6. The absorbent article according to claim 4 or 5, wherein
the second layer includes auxiliary groove portion corresponding portions at positions overlapping with the auxiliary groove portions in the thickness direction, and
an average density of the super absorbent polymer particles included in the auxiliary groove portion corresponding portions is smaller than the average density of the super absorbent polymer particles included in each of the plurality of base portion corresponding portions.

7. The absorbent article according to claim 6, wherein
the average density of the super absorbent polymer particles included in the auxiliary groove portion corresponding portions is smaller than the average density of the super absorbent polymer particles included in each of the plurality of base portions.

8. The absorbent article according to any one of claims 5 to 7, wherein
an interval of the auxiliary groove portions adjacent in the short length direction is 10 to 60 % of an interval of the plurality of main groove portions adjacent in the short length direction.

9. The absorbent article according to any one of claims 1 to 8, further comprising a core wrap which is formed by tissue in at least the non - skin facing surface of the absorbent core, wherein
the core wrap is disposed so as to protrude toward the skin facing surface side, in each of the plurality of main groove portions.
